# EUROPEAN PATENT SPECIFICATION

(11) **EP 3 041 759 B1**
(45) Date of publication and mention of the grant of the patent: **22.07.2020**
(21) Application number: 14766764.6
(22) Date of filing: 01.09.2014
(51) Int. Cl.: G01N 1/00, B65D 25/14, B65D 33/34, A61B 10/00, B65D 81/26, B65D 33/20, B01L 3/00

(54) **BAGS FOR TRANSPORT OF SPECIMENS AND A METHOD OF FORMING SUCH BAGS**
BEUTEL ZUM TRANSPORT VON PROBEN UND VERFAHREN ZUR HERSTELLUNG SOLCHER BEUTEL
SACS POUR LE TRANSPORT D'ÉCHANTILLONS ET PROCÉDÉ DE FORMATION DE TELS SACS

(30) Priority: 02.09.2013 GB 201315567
(43) Date of publication of application: 13.07.2016
(73) Proprietor: Coveris Flexibles UK Limited, Pinchbeck Spalding Lincolnshire PE11 3ZN (GB)
(72) Inventor: LUFFMAN, David, Hartlepool TS24 0RH (GB)
(74) Representative: Foot, Paul Matthew James
(86) International application number: PCT/GB2014/052642
(87) International publication number: WO 2015/028825

(56) References cited:
- EP-A1- 2 143 386
- WO-A1-2006/089297
- DE-A1-102010 010 682
- GB-A- 2 400 361
- US-A- 3 678 812
- US-A- 4 509 196
- US-A- 5 816 709
- US-A- 5 845 769

## Description

This invention relates to bags suitable for transporting specimens, such as medical samples, and to methods of making such bags. The invention also relates to a kit comprising sterilised bags sealed inside a sterilised environment and to a method of producing such kits.

An example of a bag for transporting medical samples is set out in GB2461741 which sets out the purpose of such bags in transferring containers which hold a specimen of bodily fluid to a laboratory for analysis. The bag assembly of GB2461741 comprises a backing affixed to a bag formed from two sheets of liquid impermeable plastics material, bonded together, and an absorbent pad, bonded to an inner surface of the bag. The backing, which may be paper, comprises two layers forming a pouch. A method is also disclosed, which includes depositing a pad onto an exposed surface of a first plastic web before laying the second web onto the first web, applying adhesive to the backing, and also printing on the backing.

Such a bag thus complies with legislation requiring bags for transporting medical samples to contain a pad capable of absorbing 40ml of liquid in the event of spillage from the container into the bag.

DE 102010010682 A1 discloses a transport case for transporting blood samples, which is divided into four compartments. The case is formed from two outer layers and two absorbent layers inside the outer layers. The absorbent layers extend across the entire width of the transport case. All four layers are welded together at the sides and at three locations intermediate the sides to form the case and the compartments. US5816709 shows a travel bag with a ziplock-type seal. US5845769 discloses a storage bag with a soaker pad.

This invention seeks to provide an improved bag and an improved method of forming a bag.

In accordance with a first aspect, the invention provides a bag for transporting specimens comprising a pocket for receiving a container containing a specimen and a closure for closing the pocket, wherein the pocket is formed from a liquid impervious material and has a closed end, an open mouth, side edges extending between the closed end and the open mouth, and two opposed inner surfaces, the bag further comprising a liner of absorbent material lining at least part of both opposed inner surfaces of the pocket at the closed end of the pocket, characterised in that the absorbent liner is narrower than width of the pocket from one side edge to the other and is spaced inwardly from each side edge, and the opposed surfaces of the liquid impervious material are joined directly to one another along the side edges to form the pocket.

The closure is a tamper evident closure having tamper evident features.

The closure is provided by a security tape bonded to the outer surface of one side of the pocket in the region of the mouth of the pocket; the security tape having an adhesive surface for closing the mouth of the pocket arranged such that when the closure is closed, the adhesive surface extends beyond the mouth of the pocket in all directions. More preferably the two opposing inner surfaces of the pocket are bonded together at their edges so that the mouth of the pocket does not extend to the sides of the pocket. Such arrangements reduce the possibility of leakage from the mouth of the bag.

Preferably, the opposed surfaces of the liquid impervious material are joined to one another along the side edges by heat seals.

In at least its preferred embodiments, because the absorbent liner does not extend all the way across the pocket from one side edge to the other, the opposed surfaces of the liquid impervious material can be joined directly to one another along the side edges. This firstly provides a more reliable seal, since only two layers of the same material are joined together as opposed to multiple layers of different materials as in DE 102010010682 A1 discussed above. Secondly, in prior art bags such as those shown in DE 102010010682 A1 where the absorbent liner extends all the way across the pocket, any leakage within the bag can wick to the edges of the bag, thus contaminating the edges of the bag and risking leakage and contamination of the external environment. Furthermore, external fluids can wick inwards from the side edges into the pocket, risking contamination of the sample within the bag. These issues are avoided with the present invention.

At least in preferred embodiments, there is provided a bag for transport of specimens, such as medical samples, comprising: a pocket for receiving a container containing a specimen and a closure for closing the pocket; the pocket having a closed end and an open mouth, being formed of a liquid impervious material and having two opposed inner surfaces; characterised by a liner of absorbent material lining at least part of both opposed inner surfaces of the pocket at the closed end of the pocket.

Whilst the provision of a pad of absorbent material as disclosed in the prior art complies with the relevant regulation, a liner which lines at least part of both inner surfaces of the pocket at its closed end will provide better protection if the container is broken since liquid will tend to flow towards the closed end at the bottom of the bag (since the bag will usually be transported and stored upright) and will be absorbed more quickly, reducing the chances of escape.

Broken sample containers can have sharp edges which can cut the liquid impervious material of prior art bags allowing liquid to escape. Accordingly, at least in preferred embodiments of the invention, the liner may cover a substantial portion of the inside surfaces of the bag and thereby present a barrier between the sharp edges and the surfaces of the pocket reducing the likelihood of cutting the bag and also improving the likelihood of absorbing the liquid sample if the bag is cut. Such bags are particularly suited for transport of medical samples, for transfer between hospital/surgery and laboratory for medical testing, but could also be suitable for transporting specimens from human or animal sources for testing for doping, or food testing, or transporting materials for forensic investigation.

Alternatively, if visibility of the sample inside is an important requirement, the absorbent liner may cover a smaller proportion of the inside surfaces of the bag so that, when the liquid impervious material is transparent, the sample may be more easily seen. The absorbent material itself will generally not be transparent due to the properties of the material(s) from which it is made, however the absorbent liner may be transparent or translucent.

In preferred embodiments, the absorbent liner is made from a fibrous material such as a woven or nonwoven material. The material may comprise or include cotton fibres, cellulosic fibres such as wood pulp fluff, or polymeric fibres. Preferably, the absorbent liner is made from spunbond polypropylene. In addition, the absorbent liner may also include superabsorbent polymer material.

Preferably the liner is provided by a folded sheet of absorbent material and preferably the fold in the liner is at the closed end of the pocket.

Alternatively, the liner is provided by two sheets of absorbent material each attached to a respective inner surface of the pocket. Two sheets are pinched together by the bag at the closed end in order to line the bottom surface, as well as at least part of the inner surfaces.

Preferably the closed end of the pocket is provided by a fold in the liquid impervious material. Providing a fold at the closed end, especially in combination with a folded sheet of absorbent material allows a preferential method to be used to form the bags from a single sheet of liquid impermeable material and for a single folding action to form the closed end of the bag and the fold in the liner.

Preferably the liner extends across at least 75% of the width of the inner surfaces between the sides of the pocket. More preferably the liner extends across at least 95% of the width of the inner surfaces between the sides of the pocket.

Preferably the liner extends from the closed end of the pocket to at least 50% of the height of the inner surfaces towards the open mouth of the pocket. More preferably the liner extends from the closed end of the pocket to at least 75% of the height of the inner surfaces, towards the mouth. As mentioned above, ensuring a substantial portion of the inside surface of the bag is covered by the liner, in line with these preferred features, improves the prospect of complete absorption (even if the bag is not necessarily upright) and reduces the likelihood of a broken container cutting the bag.

Preferably the tamper evident features include a patterned release layer, and/or a coating ink layer optionally applied over the release layer, and/or a thermochromic ink preferably applied in a region away from the release layer and coating ink layer, and/or an adhesive.

Preferably a pouch is provided for receiving documents relating to the specimen. More preferably the pocket and the pouch are formed from a single web with at least two folds.

Preferably the bag is printed with information for identifying the specimen.

Preferably the liquid impervious material is plastics material and preferably the liquid impervious material is transparent or translucent.

In another aspect of the invention, there is provided a kit comprising a sterile bag for transport of specimens such as medical samples, or a predetermined number of sterile bags for transport of specimens; the bag or bags including tamper-evident features and being individually packaged, or packaged in the predetermined number within a sterile environment.

Such a kit offers improvements in terms of sterility. Specimens for testing, such as medical samples, or indeed food etc. may be sensitive to microbes which could be found on or in the tamper evident bags used to transport them. Microbes could get into the bags during transport, or in storage facilities outside clean rooms in the facility where the specimen is packaged. The provision of a sterile bag packaged in its own sterile environment means that the bag, in particular the inside of the bag, will not contain contaminants and the package can be opened, the specimen placed in the bag, and the bag closed without the possibility of the bag becoming contaminated.

Sometimes there will be a requirement for several specimens to be separately packaged at the same time, hence the possibility of providing a predetermined number of bags packaged within the same sterile environment. The predetermined number could be 5, 10, or 20, for example.

Preferably the sterile environment is provided by a sachet surrounding the sterile bag or bags.

Preferably the complete kit is sterilised by gamma radiation. Sterilisation by gamma radiation can ensure that the sachet and its contents including the inside of the bag will all be sterilised at once and throughout.

In accordance with a further aspect, the present invention provides a method of forming a bag for transport of specimens comprising: advancing a web of liquid impervious material; depositing sheets of absorbent material at spaced intervals on the web of liquid impervious material; folding the web of liquid impervious material along its length, so as to provide two opposing surfaces sandwiching the sheets between them; cutting the web between the sheets of absorbent material to divide the web into discrete sections in which the absorbent sheet is narrower than the width of the section from one side edge to the other and is spaced inwardly from each side edge; and joining the opposed surfaces of the liquid impervious material directly to one another along the side edges to form a pocket with two closed sides, one closed end and an open mouth.

Preferably, the opposed surfaces of the liquid impervious material are joined to one another along the side edges by heat sealing.

Another aspect of the invention provides a method of forming a bag for transport of specimens, such as medical samples, comprising: advancing a web of liquid impervious material; depositing sheets of absorbent material at intervals on the web of liquid impervious material; folding the web of liquid impervious material along its length, so as to provide two opposing surfaces sandwiching the sheets between them; cutting the web between the sheets of absorbent material; and bonding two edges of the web to provide a pocket with two closed sides, one closed end and an open mouth.

The method is used to produce bags as defined above.

Such a method is advantageous in terms of reducing the number of bonding steps required and number of rolls of liquid impervious material required for manufacture of a bag for transporting medical samples.

Preferably the step of depositing sheets of absorbent material includes the step of adhering the sheets to the web. Adhesion to the web, for example by applying, from a web of backing material, sheets which are provided with an adhesive, will ensure that the sheets stay in position, e.g. lining the bottom of the finished bag.

Preferably the method includes a step of applying adhesive to the sheets in order to adhere the sheets to the web.

Preferably the step of folding the web of liquid impervious material causes simultaneous folding of the sheets such that each sheet forms a liner with one surface adjacent each of the two inner surfaces of the folded web.

Preferably the step of depositing sheets of absorbent material comprises depositing sheets of absorbent material in pairs. Preferably each pair of sheets of absorbent material is deposited side by side either side of the line about which the web is folded. The subsequent fold pinches the sheets together to line the closed end of the bag.

Preferably the method includes providing a closure to the bags. More preferably the closure is provided to the bags by attaching a security tape to the web of liquid impervious material.

In one preferred embodiment the steps of cutting the web and bonding at least one edge of the web are carried out simultaneously, this way one machine can carry out both steps. In another preferred embodiment the step of bonding the edges of the web to form pockets is carried out prior to cutting the web to form the bags, by this method, the bonding can be conducted before a tamper evident security tape is attached to the web.

A further aspect of the invention provides a method of forming a kit for packaging specimens for transport comprising: providing a bag, or a predetermined number of bags, the bag or bags including tamper evident features; and individually packaging the bag or bags in a sterile environment.

Preferably the sterile environment is provided by a sachet.

Preferably the method includes the steps of advancing a layer of backing material, depositing each bag, or each predetermined number of bags on the backing material at intervals; applying a further overlying layer of material over the bag or bags; sealing the overlying layer of material to the backing material; and cutting the layers of material to provide a sachet.

Preferably the method includes first individually packaging the bag, so as to provide a kit then sterilising the kit.

Another preferred embodiment includes the steps of providing a predetermined number of kits inside a further container and sterilising the plurality of kits inside the container.

Preferably a plurality of containers containing a plurality of kits are sterilised simultaneously.

Preferably sterilisation is carried out by gamma irradiation.

Preferably the resulting kit is as defined above.

Preferably the method further comprises the steps of forming the bags by the methods set out above.

Some embodiments of the invention will now be described by way of example with reference to the accompanying drawings, in which:
Figure 1 shows a perspective view of an apparatus for producing bags and bags so produced;
Figure 2 shows a perspective view of an apparatus for producing bags according to a second embodiment of the invention and bags so produced;
Figure 3 shows in detail a perspective view of a bag produced by the apparatus shown in figures 1 and 2;
Figure 4 shows a plan view of part of the bag shown in figure 3;
Figure 5 shows in plan another part of the bag of figure 3;
Figure 6 shows apparatus for forming kits comprising the bags of figure 3 inside sachets and the apparatus for sterilising such kits.
Figure 7 shows a perspective view of another bag.

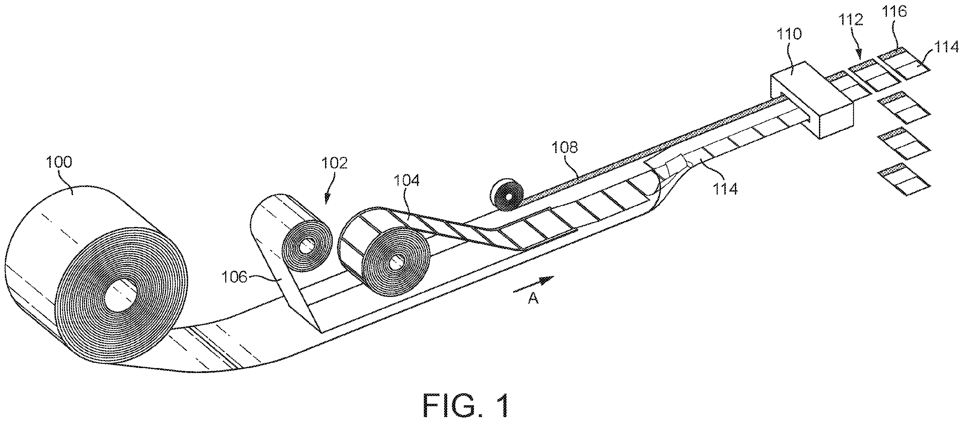

Referring to the figure 1, a web of liquid impervious material 100, in this embodiment transparent polythene, can be seen advancing from a roll in the direction of arrow A. For simplicity, the rollers etc. used to advance the web are not shown, nor is any printer or printing on the web - in practice, bar-codes, numbers and space for additional information such as "to", "from", "sample", "batch", "date/time" and "additional information" will be printed on either the inside or outside of the material. In this embodiment the web is approximately twice the width of the height of a finished bag, of course bag size is dependent on its use, but a typical final bag will be about 200mm long and therefore the web 100 would be about 400mm wide.

A machine 102 for applying sheets of absorbent material 104 is (in this embodiment) positioned above the advancing web 100. Once again, the details of the machine 102, such as rollers, supports, motors and controllers are not shown. The sheets of absorbent material 104 are supplied at spaced intervals on a roll of backing material 106. Alternatively, the sheets could be provided without spacing or even in the form of a continuous web, and conventional cut-and-place technology used to apply the absorbent sheets at spaced intervals to the web 100. The backing material 106 and sheets 104 advance in the same direction (A) as the web 100 (at the same rate) and, as the absorbent sheets are applied to the web at spaced intervals, the backing material 106 turns back on itself through an acute angle in the region adjacent the advancing web of impervious material 100 and is collected on a roll. The sheets of absorbent material 104 are thereby deposited at short intervals onto the web. Each sheet 104 of this embodiment is rectangular and is deposited on the web with its long edge perpendicular to the length of the advancing web 100 and its centreline parallel to and roughly coaxial with the centreline of the web 100 on its longitudinal axis.

Although not shown, the absorbent sheets 104 can be adhered to the surface of the web 100 by application of glue, either to the underside of the sheets 104 or the upper surface of the web 100.

A security tape 108 is shown schematically being bonded to one edge of the advancing web 100, this may be, for example, by heat sealing.

The web of liquid impervious material 100 is then shown being folded along its length, so as to provide two opposing surfaces (formerly the upper surface of the web) sandwiching the sheets of absorbent material 104 between them. It can be seen that in this embodiment, the folding action also folds the sheets 104 about their centreline, so that each sheet forms a liner 114 with one surface adjacent each of the two inner surfaces of the folded web 100.

Thus folded, the web 100 containing liners 114 of absorbent material 104 enters a heat-sealing and cutting machine 110 which cuts the web 100 transversely in the region of the intervals between the sheets of absorbent material 104 and heat-seals the edges where the web is cut and where no absorbent material 104 is present, so as to provide a pocket with two closed sides, one closed end and an open end, to which the security tape 108 is attached, thus forming a bag for transport of medical samples 112 including a liner 114 of liquid absorbent material. The heat-seal may extend very close to the sides of the liner 114 so as to leave little or no gap at the edges, which will serve to hold the edges of the absorbent liner together.

Alternative bonding methods to heat sealing may be employed to form the pocket, provided the resulting bonds provide a continuous, leak-proof seal. Other suitable bonding methods include ultrasonic bonding and adhesive bonding.

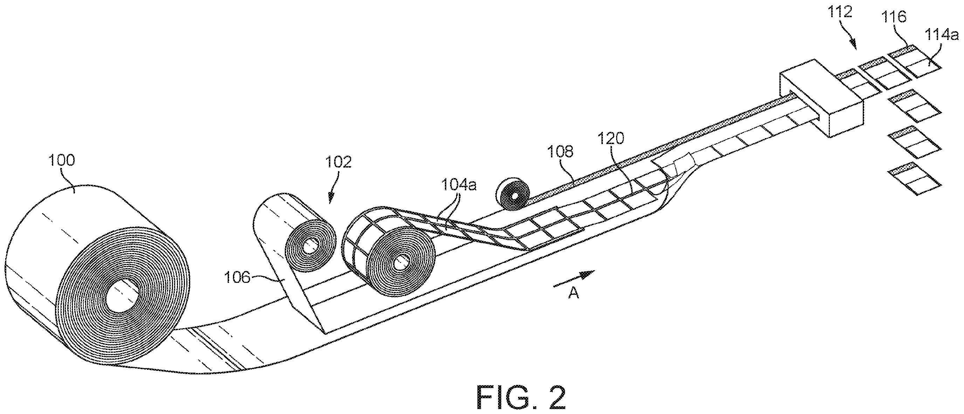

An alternative embodiment is shown in figure 2, in which like figures represent like features, in this embodiment, the machine 102 for applying sheets of absorbent material 104a carries a roll of backing material 106 on which sheets of absorbent material 104a are arranged in pairs.

Each pair of sheets of absorbent material 104a is separated longitudinally from the next pair by an interval and each member of the pair of sheets 104a is separated from the other member by a small gap 120 running along the centreline of the roll of backing material 106. Each pair of sheets of absorbent material is adhered to the web of liquid impervious material 100 with the gap 120 coaxial with the line about which the web is folded. Accordingly, when the web 100 is folded, cut and heat-sealed, each member of the pair of absorbent sheets 104a is adhered to an inside surface of the finished bag 112 forming a liner 114a.

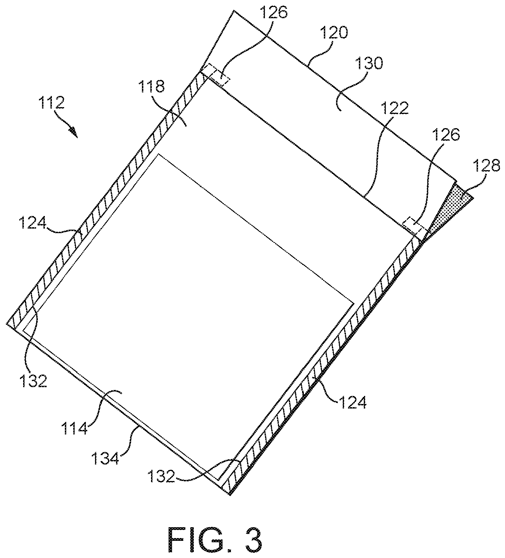

As shown in figure 3, the bags 112 produced by the process described above include a pocket 118 into which a sample can be placed, and a closure 120 formed by an adhesive flap 128 made up of the cut tape 116 and an opposing non-adhesive flap 130 formed from a portion of the web 100 which extends beyond the open end of the pocket 118. Heat seals 124 extend along the length of the parallel side edges of the pocket, defining its sides 132, whilst a block heat weld 126 extends inwardly from the sides 132 of the pocket 118 to reduce the width of the mouth 122 of the pocket 118 to reduce the prospect of leakage
The liner 114 extends substantially to the sides 132 and the closed end 134 of the pocket such that the edges are pinched together by the sides 132 of the bag 112 and similarly the opposing surfaces at the end 134 of the bag 112 are pinched together.

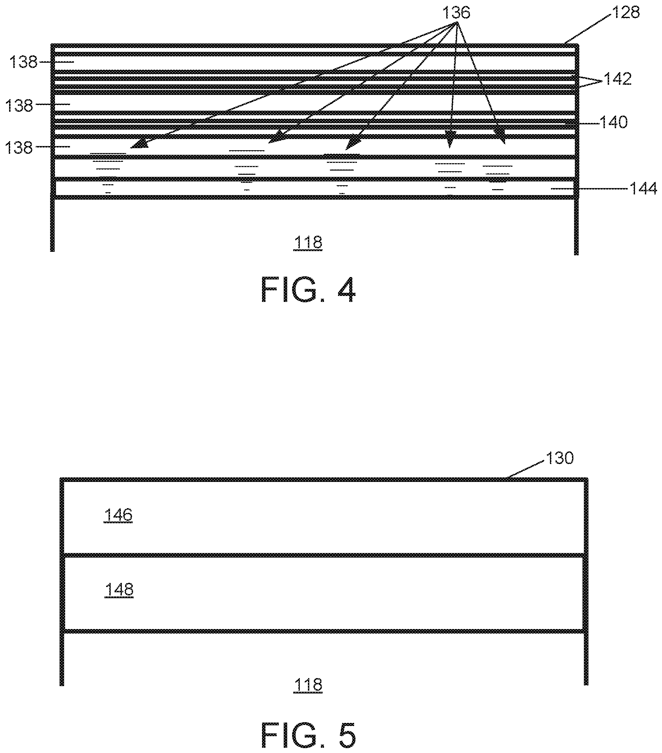

The liner 114 extends from the closed end 134 of the pocket 118 to at least 50% of the height of the inner surfaces towards the mouth 122 of the pocket 118, in this example, extending about 60% of the way to the mouth and in other preferred examples (not shown) extending even further, 90%, 95%, or as close to the block heat weld 126 as possible without preventing the welding of the two opposed surfaces of the pocket 118. An adhesive flap 128 made of a tape that can be of known construction and, as shown in figure 4 has certain the tamper-evident features on the interior surface, which is also described in European Patent No. 1860038. In this embodiment of the invention, the base material forming the flap 128 is transparent and a (pseudo-random) pattern of triangles 136 is printed on the internal surface of the tape in the region 144 where the tape is heat-sealed to the web 100, such that it can be seen externally. Three horizontal release-patterned strips 138 span the width of the adhesive flap 128 leaving upper and lower horizontal spaces between them.

The three horizontal release-patterned strips 138 each have a pattern (preferably displaying a message such as "STOP") of silicone acrylate forming a discontinuous release layer on the base material, and a layer of opaque ink coating this layer or a similar arrangement if this type, such as those described in European Patent No. 0493465. The effect of this arrangement is that if the sealed closure is peeled apart, the ink will be left on the base material in the intervening spaces left by the release pattern, whereas the ink will come away from the interior surface where the silicone acrylate is printed as it will adhere more strongly to an overlying adhesive.

In the lower space between two of the three horizontal release-patterned strips 138 a thermochromic strip 140 is printed with a thermochromic ink such as the type described in British Patent No. 2270857, preferably the ink changes from being transparent, translucent or light coloured to a bright colour, and more preferably the ink is arranged in a pattern, for example a series of words spelling "tampered" or "stop" thus on application of heat (or on cooling) the change in colour will be clearly visible through the transparent/translucent base material. The adhesive flap 128 has a final top coat of adhesive covering all the features, except for two non-adhesive strips 142 formed in the upper space between the two release-patterned strips, thus forming a discontinuous pattern. A foil release layer (not shown) covers the adhesive.

The portion of the bag 112 facing the security tape and forming the non-adhesive flap 130 is shown in figure 5 and is also provided with tamper evident features on its interior surface. This non-adhesive flap 130 is divided into two sections, an upper section 146, nearest the top of the bag and a lower section 148 closer to the mouth of the bag, each of which span the width of the bag 112, and both of which are coated in solvent detecting ink, which may change colour, or dissolve on contact with a solvent. In a most preferred embodiment, the upper section 146, facing the non-adhesive strips 142 on the interior surface of the adhesive flap 128, is coated in a solid area of solvent detecting ink and the lower section 148 is a layer of solvent detecting ink in a pattern, most preferably spelling a word, such as "void". The function of the patterned ink is to smudge on contact with a solvent whereas the combination of a solid area of solvent active ink opposite the non-adhesive strips 142 are intended to be accidentally contacted with adhesive, if someone attempts to tamper with the bag 112 by applying a layer of weak adhesive to give the appearance of closing the bag 112, but leave it not tightly closed, so that it can later be opened. In this case weak adhesive applied to the non-adhesive strips 142 will cause the opposing upper section 146 of the non-adhesive flap 130 to enter into a more intimate relationship with the non-adhesive strips 142 thus changing the appearance of the seal in that portion as the colour of the ink on the upper section 146 will be more visible through the transparent/translucent base material of the tape 108.

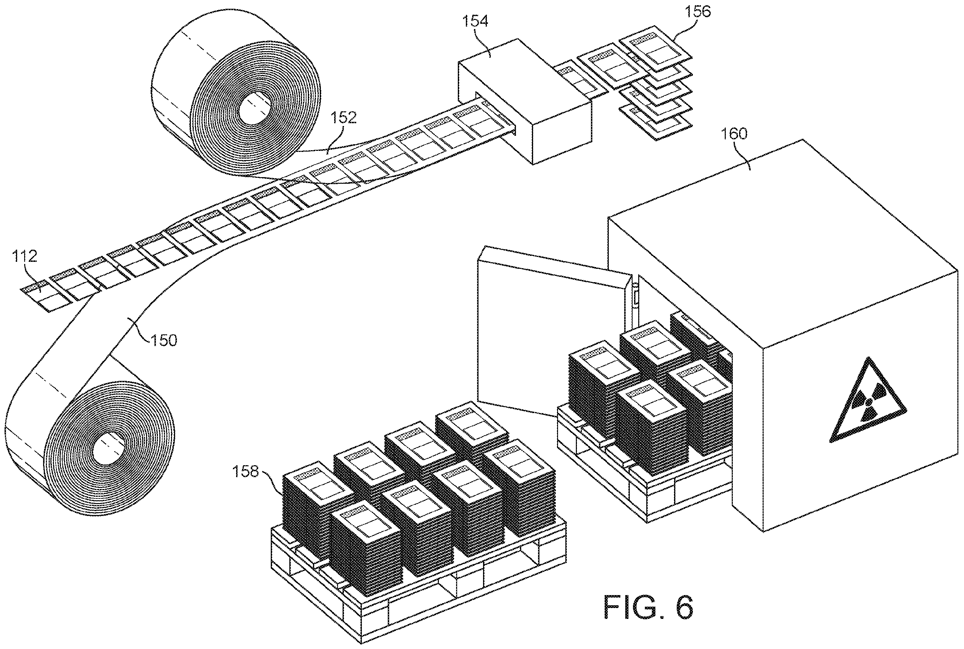

As shown in figure 6, finished bags 112 are laid at intervals on an advancing web of backing paper 150 and sandwiched between that paper and a further web of transparent plastics material 152 which is applied on top of the backing paper. Thus sandwiched between the two webs 150, 152, the finished bags 112 are passed through a machine 154 which heat seals the webs 150, around each individual bag 112 and cuts the web transversely between each bag, so as to provide individually packaged bags sealed in a sachet 156 made up of the backing paper 150 and the overlying web of transparent plastics material 152, which is suitable to maintain a sterile environment around the bag 112.

The sachets 156 are then stacked in stacks 158, placed into a further container, such as a box (not shown), palletised and introduced to an irradiation chamber 160 where they are exposed to gamma radiation. This is an effective way to sterilise the bags 112 and the environment surrounding them, such that they can be supplied individually packaged to hospitals etc. ensuring that the bags 112 within the sterilised environment will not include any microbes which could contaminate samples introduced into the bags 112.

In use, kits made up of a bag 112 for transport of medical samples, within a sachet 156 will be supplied to a hospital, surgery etc., the sachet will be opened by a user and the bag 112 for transport of medical samples opened by pulling apart the flaps 128, 130. This will open the mouth 122 of the bag, and draw apart the opposing inner surfaces of the liner - adhesion to the inner surfaces of the pocket improving the reliability of opening the space between the inner surfaces of the liner 114. A medical specimen housed in a suitable container will then be inserted into the bag 112, such that it is between the two opposing inner surfaces of the liner 114, the foil release layer (not shown) will be pulled off the adhesive flap 128, excess air expelled from the bag 112 and the adhesive flap closed against the non-adhesive flap 130 to provide a tamper-evident seal.

Optionally, the details of the sample can be entered by hand on an appropriate place on the bag, or if provided on printed material can be attached to the bag, placed in the bag, or with a suitable modification can be placed within an additional document pouch provided in the bag 112. The bags could also be provided with a detachable portion, for example as set out in WO2012/052720, on which a separable barcode could be provided, with an identical barcode provided on the bag, in order that the contents of the bag can be associated with a particular bag, and the movement of the bag can be traced. The barcode could alternatively be provided on a label (with a detachable portion) and of course, alternatives to ordinary bar codes, such as 2D barcodes, or RFID tags.

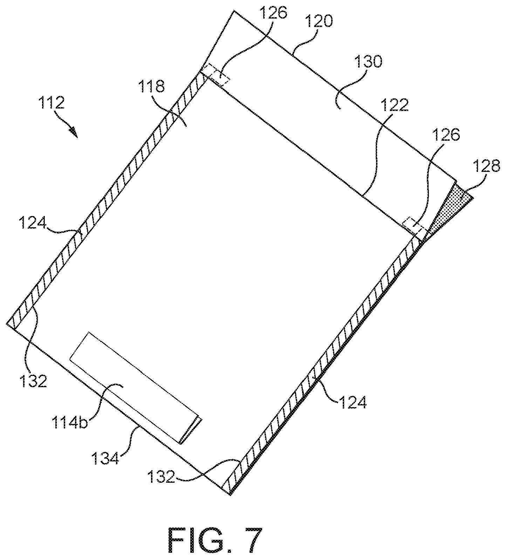

Another bag 112 produced by the process described above, but with a smaller liner 114b of highly absorbent material is shown in figure 7. As with the previous embodiment this bag 112 includes a pocket 118 into which a sample can be placed, and a closure 120 formed by an adhesive flap 128 made up of the cut tape 116 and an opposing non-adhesive flap 130 formed from a portion of the web 100 which extends beyond the open end of the pocket 118. Heat seals 124 extend along the length of the parallel side edges of the pocket, defining its sides 132, whilst a block heat weld 126 extends inwardly from the sides 132 of the pocket 118 to reduce the width of the mouth 122 of the pocket 118 to reduce the prospect of leakage

The smaller liner 114b is rectangular and has its longer sides parallel to the mouth 122 and the closed end 134 of the bag 112. By such an arrangement, this liner 114b despite being fairly small extends across a significant part of the closed end 134 of the bag 112 between the sides 132.

However, this smaller liner 114b extends from the closed end 134 of the pocket 118 to much less than 50% of the height of the inner surfaces towards the mouth 122 of the pocket 118, in this example, extending only about 15% of the way to the mouth. The closed end 134 of the bag is lined by this smaller liner 114b, so although the benefits in terms of reducing the chances of cutting the bag are diminished compared to the earlier examples, the portion of the bag where leaking fluid would accumulate is protected and such fluid will be swiftly absorbed.

Of course numerous modifications and alterations to the embodiments may be envisioned by those skilled in the art. For example, whilst the examples show the sheets of absorbent material being placed on top of the upper surface of the web, then the upper surface being folded back on itself, the sheets may be deposited on the underneath surface and the web then passed over an A-frame so as to fold the web along its length, such that the underneath surfaces form the inner surfaces of the bag, and simultaneously change the direction of travel. Likewise, instead of bonding a separate tape to the pocket to form the closure of the bags, the tamper evident features could all be applied to the surface of the bag itself in that region, as described for example in WO2012/052720. On a similar note, while certain specific details are suggested for printing on the bag, these are by no means limited; while the detailed description of most preferred embodiments refers to a machine 102 for applying sheets of absorbant material 104 from a roll, these could be applied from a fan-folded backing sheet, could be applied by a pick and place robot and could be of various alternative shapes, such as square, diamond or circular and if, as preferred, they are rectangular, they could alternatively be oriented with their long edge paralell to the length of the advancing web. The absorbant pads could even be cut from a roll to a predetermined length, picked and placed by a robot in a preprogrammed position and retained by suitable means, to simplify the starting materials, so that precut shapes on backing material is not required.

Moreover, while the absorbent sheets 104 are described in the preferred embodiment as being glued to the web 100, they need not be adhered or otherwise attached to the inside surfaces, and if they are attached, they could be retained using alternative permanent or temporary methods, such as static charge, aqueous surface tension, or ultransonic welding.

Whilst the tamper evident features of the invention are described with reference to EP1860038, obviously alternatives could be used.

Although the sachet is described as being made from a paper backing material and a front layer of polythene, alternative materials could be used for each layer. Both could be paper/polythene, for example and other materials such as Tyvek (RTM) polypropylene could replace either layer.

Other changes may be made, such as packaging a plurality, e.g. 5 or 10 bags 112 inside each sachet 156, and packaging the stacks of sachets in boxes or the like before sterilisation by gamma radiation.

## Claims

1. A bag (112) for transporting specimens comprising a pocket (118) for receiving a container containing a specimen and a closure (120) for closing the pocket (118), wherein the pocket (118) is formed from a liquid impervious material and has a closed end (134), an open mouth (122), side edges (132) extending between the closed end (134) and the open mouth (122), and two opposed inner surfaces, the bag (112) further comprising a liner (114) of absorbent material lining at least part of both opposed inner surfaces of the pocket (118) at the closed end (134) of the pocket, wherein the absorbent liner (114) is narrower than width of the pocket (118) from one side edge (132) to the other and is spaced inwardly from each side edge (132), and the opposed surfaces of the liquid impervious material are joined directly to one another along the side edges (132) to form the pocket;
**characterised in that** the closure (120) is a tamper evident closure having tamper evident features;
the closure (120) is provided by a security tape (116) bonded to the outer surface of one side of the pocket (118) in the region of the mouth (122) of the pocket; the security tape (116) having an adhesive surface for closing the mouth (122) of the pocket arranged such that when the closure (120) is closed, the adhesive surface extends beyond the mouth (122) of the pocket in all directions; and
the two opposing inner surfaces of the pocket (118) are bonded together at their edges so that the mouth (122) of the pocket does not extend to the sides of the pocket (118).

2. A bag (112) according to claim 1, wherein the opposed surfaces of the liquid impervious material are joined to one another along the side edges by heat seals (124).

3. A bag (112) according to claim 1 or 2 wherein the liner (114) is provided by a folded sheet of absorbent material, preferably wherein the fold in the liner (114) is at the closed end (134) of the pocket.

4. A bag (112) according to claim 1 or 2 wherein the liner (114) is provided by two sheets of absorbent material each attached to a respective inner surface of the pocket.

5. A bag (112) according to any of the preceding claims wherein the liner (114) extends across at least 75% of the width of the inner surfaces between the sides of the pocket, preferably wherein the liner (114) extends across at least 95% of the width of the inner surfaces between the sides of the pocket.

6. A bag (112) according to any of the preceding claims wherein the liner (114) extends from the closed end (134) of the pocket to at least 50% of the height of the inner surfaces towards the open mouth (122) of the pocket, preferably wherein the liner (114) extends from the closed end (134) of the pocket to at least 75% of the height of the inner surfaces, towards the mouth (122).

7. A bag (112) according to any preceding claim wherein the tamper evident features include a patterned release layer (138), and/or a coating ink layer optionally applied over the release layer, and/or a thermochromic ink (140) preferably applied in a region away from the release layer and coating ink layer, and/or an adhesive.

8. A kit comprising a bag (112) for transporting specimens in accordance with any preceding claim, wherein the bag is sterile, includes tamper-evident features and is individually packaged within a sterile environment, or a plurality of bags (112) for transporting specimens in accordance with any preceding claim, wherein the bags are sterile, include tamper evident features and are packaged together within a sterile environment.

9. A method of forming a bag (112) according to any one of claims 1 to 8 for transport of specimens comprising:
advancing a web (106) of liquid impervious material;
depositing sheets (104) of absorbent material at spaced intervals on the web (106) of liquid impervious material;
folding the web (106) of liquid impervious material along its length, so as to provide two opposing surfaces sandwiching the sheets between them;
cutting the web (106) between the sheets (104) of absorbent material to divide the web (106) into discrete sections in which the absorbent sheet (104) is narrower than the width of the section from one side edge to the other and is spaced inwardly from each side edge;
joining the opposed surfaces of the liquid impervious material directly to one another along the side edges to form a pocket (118) with two closed sides, one closed end (134) and an open mouth (122); and
providing a closure (120) to the bag (112);
**characterised in that** the closure (120) is provided to the bag by attaching a security tape (108) to the web (106) of liquid impervious material.

10. A method according to claim 9, wherein the opposed surfaces of the liquid impervious material are joined to one another along the side edges (132) by heat sealing.

11. A method according to claim 9 or 10 wherein the step of depositing sheets (104) of absorbent material includes the step of adhering the sheets to the web (106).

12. A method according to any of claims 9 to 11 wherein a step of applying adhesive to the sheets (104) is provided in order to adhere the sheets to the web (106).

13. A method according to any of claims 9 to 12 wherein the step of folding the web (106) of liquid impervious material causes simultaneous folding of the sheets (104) such that each sheet (104) forms a liner (114) with one surface adjacent each of the two inner surfaces of the folded web (106).

14. A method of forming a kit for packaging specimens for transport comprising forming a bag (112) or a plurality of bags (112) for transport of specimens in accordance with any of claims 9 to 14, adding tamper-evident features to the bag or bags, sterilising the bag or bags, and individually packaging the bag or bags in a sterile environment.

## Patentansprüche

1. Beutel (112) zum Transportieren von Proben, umfassend eine Tasche (118) für die Aufnahme eines eine Probe enthaltenden Behältnisses und einen Verschluss (120) zum Verschließen der Tasche, wobei die Tasche (118) aus einem flüssigkeitsundurchlässigen Material gebildet ist und ein geschlossenes Ende (134), eine Öffnung (122), sich zwischen dem geschlossenen Ende (134) und der Öffnung (122) erstreckende Seitenkanten (132) und zwei einander gegenüberliegende Innenflächen hat, wobei der Beutel (112) ferner ein Futter (114) aus absorbierendem Material aufweist, das zumindest einen Teil der einander gegenüberliegenden Innenflächen der Tasche (118) an deren geschlossenem Ende (134) ausfüttert, wobei das absorbierende Futter (114) schmaler ist als die Breite der Tasche (118) von einer Seitenkante (132) zu der anderen und von jeder Seitenkante (132) nach innen beabstandet ist und wobei die einander gegenüberliegenden Flächen des flüssigkeitsundurchlässigen Materials zum Bilden der Tasche entlang der Seitenkanten (132) direkt miteinander verbunden sind;
**dadurch gekennzeichnet, dass** der Verschluss (120) ein manipulationssicherer Verschluss ist, der über Manipulationsnachweismerkmale verfügt;
dass der Verschluss (120) durch ein Sicherheitsband (116) bereitgestellt wird, das in dem Bereich der Öffnung (122) der Tasche an die Außenfläche einer Seite der Tasche (118) geklebt ist;
dass das Sicherheitsband (116) eine Klebefläche zum Verschließen der Taschenöffnung (122) aufweist, die derart angeordnet ist, dass sich die Klebefläche bei geschlossenem Verschluss (120) in allen Richtungen über die Öffnung (122) der Tasche hinaus erstreckt; und
dass die beiden einander gegenüberliegenden Innenflächen der Tasche (118) an ihren Kanten derart miteinander verklebt sind, dass sich die Öffnung (122) nicht zu den Seiten der Tasche (118) erstreckt.

2. Beutel (112) nach Anspruch 1, wobei die einander gegenüberliegenden Flächen des flüssigkeitsundurchlässigen Materials entlang der Seitenkanten durch Heißsiegel (124) miteinander verbunden sind.

3. Beutel (112) nach Anspruch 1 oder 2, wobei das Futter (114) durch einen gefalteten Bogen eines absorbierenden Materials gebildet ist, wobei der Falz in dem Futter (114) bevorzugt an dem geschlossenen Ende (134) der Tasche liegt.

4. Beutel (112) nach Anspruch 1 oder 2, wobei das Futter (114) durch zwei Bögen eines absorbierenden Materials gebildet ist, die jeweils an einer betreffenden Innenfläche der Tasche befestigt sind.

5. Beutel (112) nach einem der vorangehenden Ansprüche, wobei sich das Futter (114) über mindestens 75% der Breite der Innenflächen zwischen den Seiten der Tasche erstreckt und wobei sich das Futter (114) bevorzugt über 95% der Breite der Innenflächen zwischen den Seiten der Tasche erstreckt.

6. Beutel (112) nach einem der vorangehenden Ansprüche, wobei sich das Futter (114) von dem geschlossenen Ende (134) der Tasche bis zu mindestens 50% der Höhe der Innenflächen in Richtung auf die Öffnung (122) der Tasche erstreckt und wobei sich das Futter (114) bevorzugt von dem geschlossenen Ende (134) der Tasche bis zu mindestens 75% der Höhe der Innenflächen in Richtung auf die Öffnung (122) erstreckt.

7. Beutel (112) nach einem der vorangehenden Ansprüche, wobei die Manipulationsnachweismerkmale eine strukturierte Ablöseschicht (138) und/oder optional eine über der Ablöseschicht aufgetragene Streichfarbenschicht und/oder eine thermochrome Farbe (140), die vorzugsweise in einem von der Ablöseschicht und Streichfarbenschicht entfernten Bereich aufgetragen ist, und/oder einen Klebstoff umfassen.

8. Kit, enthaltend einen Beutel (112) zum Transportieren von Proben gemäß einem der vorangehenden Ansprüche, wobei der Beutel steril ist, Manipulationsnachweismerkmale besitzt und einzeln in einer sterilen Umgebung verpackt ist, oder enthaltend eine Mehrzahl von Beuteln (112) zum Transportieren von Proben gemäß einem der vorangehenden Ansprüche, wobei die Beutel steril sind, Manipulationsnachweismerkmale besitzen und zusammen in einer sterilen Umgebung verpackt sind.

9. Verfahren zur Herstellung eines Beutels (112) gemäß einem der Ansprüche 1 bis 8 zum Transportieren von Proben, umfassend:
den Vorschub einer Bahn (106) eines flüssigkeitsundurchlässigen Materials;
das Ablegen von Bögen (104) eines absorbierenden Materials in beabstandeten Intervallen auf der Bahn (106) des flüssigkeitsundurchlässigen Materials;
das Falten der Bahn (106) des flüssigkeitsundurchlässigen Materials entlang seiner Länge, so dass zwei einander gegenüberliegende Flächen gebildet werden, die die Bögen zwischen sich aufnehmen;
das Schneiden der Bahn (106) zwischen den Bögen (104) des absorbierenden Materials, um die Bahn (106) in einzelne Abschnitte zu unterteilen, in denen der absorbierende Bogen (104) schmaler ist als die Breite des Abschnitts von einer Seitenkante zur anderen und von jeder Seitenkante nach innen beabstandet ist;
das direkte Verbinden der einander gegenüberliegenden Flächen des flüssigkeitsundurchlässigen Materials miteinander entlang der Seitenkanten, um eine Tasche (118) mit zwei geschlossenen Seiten, einem geschlossenen Ende (134) und einer Öffnung (122) zu bilden;
das Vorsehen eines Verschlusses (120) an dem Beutel;
**dadurch gekennzeichnet, dass** der Verschluss (120) an dem Beutel vorgesehen wird, indem ein Sicherheitsstreifen (108) an der Bahn (106) des flüssigkeitsundurchlässigen Materials befestigt wird.

10. Verfahren nach Anspruch 9, wobei die einander gegenüberliegenden Flächen des flüssigkeitsundurchlässigen Materials entlang der Seitenkanten (132) durch Heißsiegeln miteinander verbunden werden.

11. Verfahren nach Anspruch 9 oder 10, wobei der Schritt des Ablegens von Bögen (104) absorbierenden Materials den Schritt des Verklebens der Bögen mit der Bahn (106) umfasst.

12. Verfahren nach einem der Ansprüche 9 bis 11, wobei ein Schritt des Auftragens eines Klebstoffs auf die Bögen (104) vorgesehen ist, um die Bögen mit der Bahn (106) zu verkleben.

13. Verfahren nach einem der Ansprüche 9 bis 12, wobei der Schritt des Faltens der Bahn (106) flüssigkeitsundurchlässigen Materials bewirkt, dass gleichzeitig die Bögen (104) gefaltet werden, so dass jeder Bogen (104) ein Futter (114) mit je einer an die beiden Innenflächen der gefalteten Bahn angrenzenden Fläche bildet.

14. Verfahren zum Herstellen eines Kits zum Verpacken von Proben für den Transport, umfassend das Bilden eines Beutels (112) oder einer Mehrzahl von Beuteln (112) für den Transport von Proben gemäß einem der Ansprüche 9 bis 14, das Hinzufügen von Manipulationsnachweismerkmalen zu dem oder den Beuteln, das Sterilisieren des oder der Beutel und das einzelne Verpacken des oder der Beutel in einer sterilen Umgebung.

## Revendications

1. Sac (112) pour le transport d'échantillons comprenant une poche (118) pour recevoir un conteneur avec un échantillon et une fermeture (120) pour fermer la poche (118)
dans lequel
la poche (118) est formée d'une matière imperméable aux liquides et elle a une extrémité fermée (134), une embouchure (122), des côtés (132) entre l'extrémité fermée (134) et l'embouchure ouverte (122) ainsi que deux surfaces intérieures opposées, la poche (112) ayant en outre un revêtement (114) réalisé comme un revêtement de matière absorbante au moins en partie sur les deux surfaces intérieures opposées de la poche (118) à l'extrémité fermée (134) de la poche,
le revêtement absorbant (114) étant plus étroit que la largeur de la poche (118) à partir d'un côté (132) vers l'autre et il est écarté vers l'intérieur à partir de chaque bord (132) et les surfaces opposées en matière imperméable aux liquides sont réunies directement l'une à l'autre le long des bords (132) pour former la poche,
sac **caractérisé en ce que**
la fermeture (120) est une fermeture inviolable ayant des caractéristiques de fermeture inviolable,
- la fermeture (120) a une bande de sécurité (116) fixée à la surface extérieure sur un côté de la poche (118) dans la région de l'embouchure (122) de la poche, la bande de sécurité (116) ayant une surface adhésive pour fermer l'embouchure (122) de la poche, cette bande étant disposée de façon que lorsque la fermeture (120) est fermée, la surface adhésive dépasse de l'embouchure (120) de la poche dans toutes les directions et les deux surfaces intérieures opposées de la poche (118) sont reliées l'une à l'autre selon leur bord de façon que l'embouchure (122) de la poche ne dépasse pas des côtés de la poche (118).

2. Sac (112) selon la revendication 1,
dans lequel
des surfaces opposées de la matière imperméable au liquide sont réunies l'une à l'autre le long de deux côtés par des thermo soudures (124).

3. Sac (112) selon la revendication 1 ou 2,
dans lequel
le revêtement (114) est réalisé par une feuille pliée de matière absorbante de préférence dans lequel le pli du revêtement (114) se trouve à l'extrémité fermée (134) de la poche.

4. Sac (112) selon la revendication 1 ou 2,
dans lequel
le revêtement (114) est réalisé par deux feuilles de matière absorbante, chacune étant fixée à la surface intérieure respective de la poche.

5. Sac (112) selon l'une quelconque des revendications précédentes,
dans lequel
le revêtement (114) s'étend sur au moins 75% de la largeur des surfaces intérieures entre les côtés de la poche, le revêtement (114) s'étend de préférence sur au moins 95% de la largeur des surfaces intérieures entre les côtés de la poche.

6. Sac (112) selon l'une quelconque des revendications précédentes,
dans lequel
le revêtement (114) s'étend à partir de l'extrémité fermée (134) de la poche jusqu'à au moins 50% de la hauteur des surfaces intérieures vers l'embouchure ouverte (122) de la poche, de préférence le revêtement (114) s'étendant à partir de l'extrémité fermée (134) de la poche jusqu'à au moins 75% de la hauteur des surfaces intérieures en direction de l'embouchure (122).

7. Sac (112) selon l'une quelconque des revendications précédentes,
dans lequel
les caractéristiques d'inviolabilité comprennent une couche amovible munie d'un motif (138) et/ou d'une couche d'encre de revêtement appliquée en option sur la couche amovible et/ou une encre thermochromique (140) appliquée de préférence dans une région éloignée de la couche amovible et de la couche d'encre de revêtement et/ou d'un adhésif.

8. Kit composé d'un sac (112) pour transporter des échantillons selon l'une quelconque des revendications précédentes,
dans lequel
le sac est stérile et comprend des caractéristiques d'inviolabilité et il est conditionné individuellement dans un environnement stérile ou encore un ensemble de sacs (112) pour transporte des échantillons selon l'une quelconque des revendications précédentes, dans lequel les sacs sont stériles, ont des caractéristiques d'inviolabilité et sont conditionnés ensemble dans un environnement stérile.

9. Procédé de formation d'un sac (112) selon l'une quelconque des revendications 1 à 8 pour transporter des échantillons consistant à :
- faire avancer une nappe (106) d'une matière imperméable aux liquides
- déposer des feuilles (104) de matière absorbante à des intervalles sur la nappe (106) de matière imperméable aux liquides
- plier la nappe (106) de matière imperméable aux liquides selon sa longueur de façon à obtenir deux surfaces opposées comprenant entre elles les feuilles ;
- couper la nappe (106) entre les feuilles (104) de matière absorbante pour diviser la nappe (106) en sections distinctes dans lesquelles la feuille absorbante (104) est plus étroite que la largeur de la section d'un côté à l'autre et est espacée vers l'intérieur à partir de chaque côté,
- réunir les surfaces opposées de matière imperméable aux liquides directement l'une à l'autre le long des côtés pour former une poche (118) avec deux côtés fermés, une extrémité fermée (134) et une embouchure ouverte (122) et
réaliser une fermeture (120) de la poche (112)
procédé **caractérisé en ce que**
la fermeture (120) est réalisée pour le sac en fixant une bande de sécurité (108) sur la nappe (106) de matière imperméable aux liquides.

10. Procédé selon la revendication 9,
selon lequel
des surfaces opposées de matière imperméable aux liquides sont réunies l'une à l'autre le long des côtés (132) par thermosoudage.

11. Procédé selon l'une des revendications 9 ou 10,
selon lequel
l'étape de dépôt des feuilles (104) de matière absorbante comprend une étape de collage des feuilles à la nappe (106).

12. Procédé selon l'une des revendications 9 à 11,
selon lequel
une étape d'application d'adhésif sur les feuilles (104) assure l'adhérence des feuilles à la nappe (106).

13. Procédé selon l'une des revendications 9 à 12,
selon lequel
l'étape de pliage de la nappe (106) de matière imperméable au liquide réalise le pliage simultané des feuilles (104) de façon que chaque feuille (104) forme un revêtement (114) avec une surface adjacente à chacune des deux surfaces intérieures de la nappe pliée (106).

14. Procédé pour former un kit d'emballage d'échantillons pour le transport comprenant les étapes consistant à
- former une poche (112) ou un ensemble de poches (112) pour transporter des échantillons selon l'une quelconque des revendications 9 à 14, ajouter les caractéristiques d'inviolabilité à la poche ou aux poches, stériliser la ou les poches et emballer individuellement la ou les poches dans un environnement stérile.
